(19) Europäisches Patentamt
European Patent Office
Office européen des brevets

(11) **EP 3 653 987 A1**

(12) **EUROPEAN PATENT APPLICATION**

(43) Date of publication:
**20.05.2020 Bulletin 2020/21**

(51) Int Cl.:
**G01B 9/02** (2006.01)          **A61B 5/00** (2006.01)
**G01J 3/28** (2006.01)          **G01J 3/02** (2006.01)
**G01B 11/24** (2006.01)

(21) Application number: **18206841.1**

(22) Date of filing: **16.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nokia Technologies Oy**
**02610 Espoo (FI)**

(72) Inventors:
• YUAN, Xin
  **New Providence, New Jersey 07974 (US)**
• WILFORD, Paul
  **Bernardville, New Jersey 07924 (US)**

(74) Representative: **Swindell & Pearson Limited**
**48 Friar Gate**
**Derby DE1 1GY (GB)**

(54) **APPARATUS AND METHOD FOR DETECTING LIGHT**

(57)     Apparatus and method for detecting light. The apparatus comprises means for splitting an input beam of light into at least a first beam of light and a second beam of light wherein the input beam of light is obtained from an optical coherence tomography arrangement. The apparatus also comprises means for modulating the first beam of light to provide a first modulated beam of light and means for modulating the second beam of light to provide a second modulated beam of light. The apparatus also comprises means for dispersing the first modulated beam of light to provide a first dispersed beam of light and means for dispersing the second modulated beam of light to provide a second dispersed beam of light. The apparatus also comprises means for detecting the first dispersed beam of light and means for detecting the second dispersed beam of light wherein the means for detecting the first dispersed beam of light and means for detecting the second dispersed beam of light are configured to convert the detected beams of light into electrical output signals.

FIG. 4

**Description**

TECHNOLOGICAL FIELD

Examples of the disclosure relate to apparatus and methods for detecting light. In particular they relate to apparatus and methods for detecting light from an optical coherence tomography arrangement.

BACKGROUND

Optical coherence tomography enables cross sectional imaging of an object such as a retina or other part of a body by detecting the light reflected from internal structures within the object.
It is useful to provide means for detecting the light from optical coherence tomography arrangements which enable a high quality image to be obtained.

BRIEF SUMMARY

According to various, but not necessarily all, examples of the disclosure there is provided an apparatus comprising: means for splitting an input beam of light into at least a first beam of light and a second beam of light wherein the input beam of light is obtained from an optical coherence tomography arrangement; means for modulating the first beam of light to provide a first modulated beam of light and means for modulating the second beam of light to provide a second modulated beam of light; means for dispersing the first modulated beam of light to provide a first dispersed beam of light and means for dispersing the second modulated beam of light to provide a second dispersed beam of light; and means for detecting the first dispersed beam of light and means for detecting the second dispersed beam of light wherein the means for detecting the first dispersed beam of light and means for detecting the second dispersed beam of light are configured to convert the detected beams of light into electrical output signals.
The means for splitting an input beam of light into at least a first beam of light and a second beam of light may be configured to split the input beam of light into more than two beams of light.
The apparatus may comprise means for modulating for each of the beams of light provided by the means for splitting an input beam of light, means for dispersing for each of the modulated beams of light and means for detecting for each of the dispersed beams of light.
The means for modulating the beams of light may comprise one or more coded apertures.
The one or more coded apertures may comprise a two dimensional coded aperture.
The means for modulating the beams of light may comprise at least a first portion having a first transparency to the beam of light and at least a second portion having a second transparency to the beam of light, the second transparency being different from the first transparency.
The first transparency and the second transparency may be wavelength dependent.
The portions of the means for modulating the input beam of light having different transparencies may be arranged in a random pattern.
The means for modulating the beams of light may be arranged to convert a three dimensional signal into a two dimensional signal.
The means for modulating the beams of light may be arranged to be moveable relative to the means for dispersing the modulated beams of light and means for detecting the dispersed beams of light.
The means for dispersing the modulated beams of light may comprise at least one of; a prism, and a grating.
The means for detecting the dispersed beams of light may comprise at least one of a: charge coupled device, and a complementary metal-oxide semiconductor sensor.
The means for detecting the dispersed beams of light may comprise a two dimensional array of sensors.
The optical coherence tomography arrangement may be arranged so that the input beam of light comprises different wavelengths of light and the different wavelengths of light provide information about different depths within the object.
According to various, but not necessarily all, examples of the disclosure there is provided an apparatus comprising: at least one beam splitter configure to split an input beam of light into at least a first beam of light and a second beam of light wherein the input beam of light is obtained from an optical coherence tomography arrangement; a first modulator configured to modulate the first beam of light to provide a first modulated beam of light and a second modulator configured to modulate the second beam of light to provide a second modulated beam of light; a first disperser configured to disperse the first modulated beam of light to provide a first dispersed beam of light and a second disperser configured to disperse the second modulated beam of light to provide a second dispersed beam of light; and a first detector configured to detect the first dispersed beam of light and a second detector configured to detect the second dispersed beam of light wherein the first detector the second detector are configured to convert the detected beams of light into electrical output signals.

According to various, but not necessarily all, examples of the disclosure there is provided a method comprising: splitting an input beam of light into at least a first beam of light and a second beam of light wherein the input beam of light is obtained from an optical coherence tomography arrangement; modulating the first beam of light to provide a first modulated beam of light and modulating the second beam of light to provide a second modulated beam of light; dispersing the first modulated beam of light to provide a first dispersed beam of light and dispersing the second modulated beam of light to provide a second dispersed beam of light; and detecting the first dispersed beam of light and detecting the second dispersed beam of light and converting the detected beams of light into electrical output signals.

The method may comprise splitting the input beam of light into more than two beams of light.

The method may comprise modulating, dispersing and detecting each of the beams of light.

BRIEF DESCRIPTION

Some example embodiments will now be described with reference to the accompanying drawings in which:

Fig. 1 illustrates an example apparatus;
Fig. 2 illustrates another example apparatus;
Fig. 3 illustrates an imaging principle;
Fig. 4 illustrates an optical coherence tomography arrangement and an apparatus;
Fig. 5 illustrates a method;
Figs. 6A to 6H illustrate example images obtained using an example apparatus; and
Fig. 7 illustrates a method of using examples of the disclosure.

DETAILED DESCRIPTION

Examples of the disclosure relate to an apparatus 101 which can be used as a detector for an optical coherence tomography (OCT) arrangement 121. The apparatus 101 provides for compressed sampling of the input beam of light and disperses different bandwidths of the beam of light. In examples of the disclosure the input beam of light is split into two or more beams of light. The two or more beams of lights can be modulated and dispersed independently of each other so as to provide a more detailed image. The image may be a high-quality image.

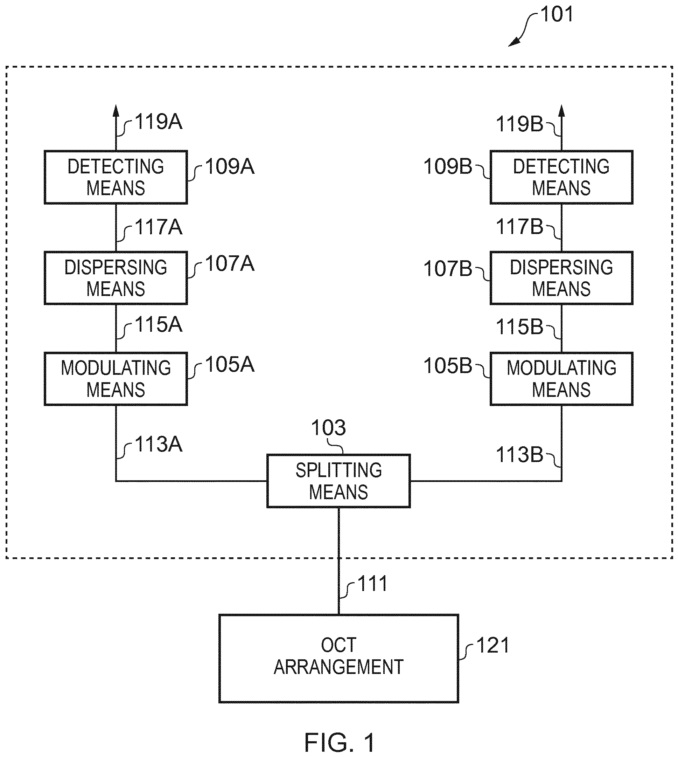

Fig. 1 schematically illustrates an example apparatus 101. The example apparatus 101 comprises means 103 for splitting an input beam of light 111 into at least a first beam of light 113A and a second beam of light 113B. The input beam of light 111 is obtained from an OCT arrangement 121. The OCT arrangement 121 may be a full-filed spectral-domain OCT arrangement 121. The apparatus 101 also comprises means 105A for modulating the first beam of light 113A to provide a first modulated beam of light 115A and means 105B for modulating the second beam of light 113B to provide a second modulated beam of light 115B. The apparatus 101 also comprises means 107A for dispersing the first modulated beam of light 115A to provide a first dispersed beam of light 117A and means 107B for dispersing the second modulated beam of light 115B to provide a second dispersed beam of light 117B. The apparatus 101 also comprises means 109A for detecting the first dispersed beam of light 117A and means 109B for detecting the second dispersed beam of light 117B wherein the means 109A for detecting the first dispersed beam of light 117A and means 109B for detecting the second dispersed beam of light 117B are configured to convert the detected beams of light into electrical output signals 119A, 119B.

The means 103 for splitting an input beam of light 111 into at least a first beam of light 113A and a second beam of light 113B may comprise one or more beam splitters.

In some examples the one or more beam splitters may be configured to split the input beam of light 111 so that each of the split beams 113A, 113B has the same intensity. In other examples the one or more beam splitters may be configured to split the input beam of light 111 so that different split beams 113A, 113B have different intensities.

The one or more beam splitters are configured within the apparatus 101 so that when the apparatus 101 is coupled to an OCT arrangement 121, an input beam of light 111 from the OCT arrangement 121 is incident, at least in part, upon the one or more beam splitters.

In the example apparatus 101 shown in Fig. 1 the first beam of light 113A is provided to a first channel and the second beam of light 113B is provided to a second channel. Each of the first channel and the second channel enable sparse sampling of the respective beam of light. Each of the first channel and the second channel comprise modulating means 105A, 105B, dispersing means 107A, 107B and detecting means 109A, 109B.

The means 105A, 105B for modulating the first beam of light 113A and the second beam of light 113B comprise one or more modulators. The modulators are configured within the apparatus 101 so that the beams of light 113A, 113B from the one or more beam splitters are incident, at least in part on the one or more modulators. In the example shown in Fig. 1 the first beam of light 113A is incident on a first modulator and the second beam of light 113B is incident on a second modulator.

The modulators enable compressed sampling of the beams of light 113A, 113B. The modulators are configured to selectively remove information from beams of light 113A, 113B so that only portions of the beams of light 113A, 113B are detected. In some examples the modulators may be configured to convert a three dimensional signal into a two dimensional signal.

The modulators may comprise any means which may be configured to spatially modulate the beams of light 113A, 113B. In some examples the modulators may comprise one or more coded apertures. The coded apertures may comprise optical masks or any other suitable type of aperture. The coded apertures may be two dimensional coded apertures or any other suitable type of apertures. In other examples the modulators could comprise a liquid crystal on silicon (LCOS) modulator, a digital micro-mirror device (DMD), or any other suitable type of modulator.

In some examples the modulators may comprise at least a first portion having a first level of transparency to the beams of light 113A, 113B and at least a second portion having a second, different level of transparency to the beams of light 113A, 113B. The different levels of transparency may allow different levels of light to pass through the different portions of the modulators. It is to be appreciated that the modulators may comprise a plurality of first portions and a plurality of second portions. In some examples the modulators may be binary modulators so that only two different transparencies are provided by the respective portions of each modulator. In other examples the modulators could be a grey-scale aperture and may comprise more than two different levels of transparency in the different portions of the modulator.

In some examples the transparency of the different portions of the modulator may be wavelength dependent. In such examples the modulation of the beams of light 113A, 113B by the respective portions of the modulators will be dependent upon the wavelengths within the beams of light 113A, 113B.

The different portions of the one or more modulators may be arranged in any suitable pattern. In some examples the respective portions of the modulators having different transparencies are arranged in a pixelated arrangement. The pixelated arrangement may comprise the respective portions of the modulators being arranged in an array of columns and rows. The rows and columns may correspond to the pixels in the detecting means 109A, 109B. The number of transparent pixels, partially transparent pixels, and non- transparent pixels may vary in different implementations of the disclosure. In some examples approximately half of the pixels could be transparent so that half of the incident area of the modulator acts to block beams of light 113A, 113B while the other half allows the beams of light 113A, 113B, or partially pass through.

In some examples the different portions of the one or more modulators having different transparencies may be arranged in a random pattern. The random pattern may be an irregular pattern. The random pattern might not be defined or arranged in relation to any specific object. In some examples the different portions of the modulator may be arranged in a pseudo random pattern. In other examples the respective portions of the modulator may be arranged in a predetermined or customised pattern. The predetermined or customised pattern may be selected according to the object or type of object that is to be imaged by the OCT system.

In some examples the modulators may be fixed in position relative to the other components of the apparatus 101. In other examples the modulators may be arranged to be moveable relative to the other components of the apparatus 101. In particular the modulators may be moveable so that the modulators can be shifted relative to the means 107A, 107B for dispersing the modulated beams of light 115A, 115B and the means 109A, 109B for detecting the dispersed beams of light 117A, 117B.

In the example shown in Fig. 1 the first modulator provides a first modulated beam of light 115A as an output and the second modulator provides a second modulated beam of light 115B as an output. The first modulator may be independent of the second modulator. In some examples the first modulator could be the same as the second modulator so that the same modulation is provided to the first beam of light 113A and the second beam of light 113B. In other examples the first modulator could be different to the second modulator so that different modulation is provided to the first beam of light 113A and the second beam of light 113B.

The example apparatus 101 shown in Fig. 1 comprises a first dispersing means 107A and a second dispersing means 107B. The first dispersing means 107A is configured within the apparatus 101 so that the first modulated beam of light 115A, or at least part of the first modulated beam of light 115A, provided by the first modulating means 105A is incident upon the first dispersing means 107A. The second dispersing means 107B is configured within the apparatus 101 so that the second modulated beam of light 115B, or at least part of the second modulated beam of light 115B, provided by the second modulating means 105B is incident upon the second dispersing means 107B.

The means 107A, 107B for dispersing the modulated beams of light 115A, 115B may comprise one or more dispersing elements. The dispersing elements may comprise any elements which cause different wavelengths of the modulated beams of light 115A, 115B to be refracted by different amounts. The one or more dispersing elements may comprise prisms, gratings or any other suitable elements.

The first dispersing means 107A may be configured to disperse the light in a first direction and the second dispersing means 107B may be configured to disperse the light in a second, different direction. In some examples the first direction could be perpendicular, or substantially perpendicular, to the second direction.

In some examples the first dispersing means 107A could be the same, or substantially the same, as the second dispersing means 107B so that the modulated beams of the light 115A, 115B are refracted by the same amounts or substantially the same amounts. In other examples the first dispersing means 107A and the second dispersing means 107B could be different so that the modulated beams of the light 115A, 115B are refracted by different amounts. In example apparatus 101 where the dispersing means 107A, 107B are different it may be necessary to perform processing on the detected signals to take into account the differences in the dispersing means 107A, 107B.

The first dispersing means 107A is configured to provide a first dispersed beam of light 117A as an output and the second dispersing means 107B is configured to provide a second dispersed beam of light 117B. The first means 109A for detecting the first dispersed beam of light 117A is configured within the apparatus 101 so that the first dispersed beam of light 117A, or at least part of the first dispersed beam of light 117A, is incident on the first means 109A for detecting the first dispersed beam of light 117A. The second means 109B for detecting the second dispersed beam of light 117B is configured within the apparatus 101 so that the second dispersed beam of light 117B, or at least part of the second dispersed beam of light 117B, is incident on the second means 109B for detecting the second dispersed beam of light 117B.

The means 109A, 109B for detecting the dispersed beams of light 117A, 117B comprise detectors. The detectors 109A, 109B may be arranged to transduce incident light into an electrical output signal. In some examples the detectors 109A, 109B may comprise charge-coupled devices, complementary metal-oxide semiconductor (CMOS) sensors or any other suitable type of sensors.

In some examples the detectors 109A, 109B may comprise two dimensional arrays of sensors. In other examples the detectors 109A, 109B may comprise linear detectors which may be scanned across a detecting plane. In some examples the first detector 109A and the second detector 109B could be the same type of detectors.

In the example apparatus 101 shown in Fig. 1 the first detector 109A provides a first output signal 119A and the second detector 109B provides a second output signal 119B. The first output signal 119A and the second output signal 119B both comprise information indicative of an object imaged by the OCT arrangement 121. However, the images that can be obtained from the output signals 119A, 119B may be different because the first dispersing means 107A and the second dispersing means 107B disperse the light in different directions. This means that different information could be comprised in the different output signals 119A, 119B.

In some examples the first output signal 119A and the second output signal 119B can be combined to provide a single output signal which represents an image of the object imaged by the OCT arrangement 121. This image obtained using two different modulating and dispersing channels may be more accurate and may comprise more information than an image obtained using a single channel. The image could be rendered on a display or other suitable user output device.

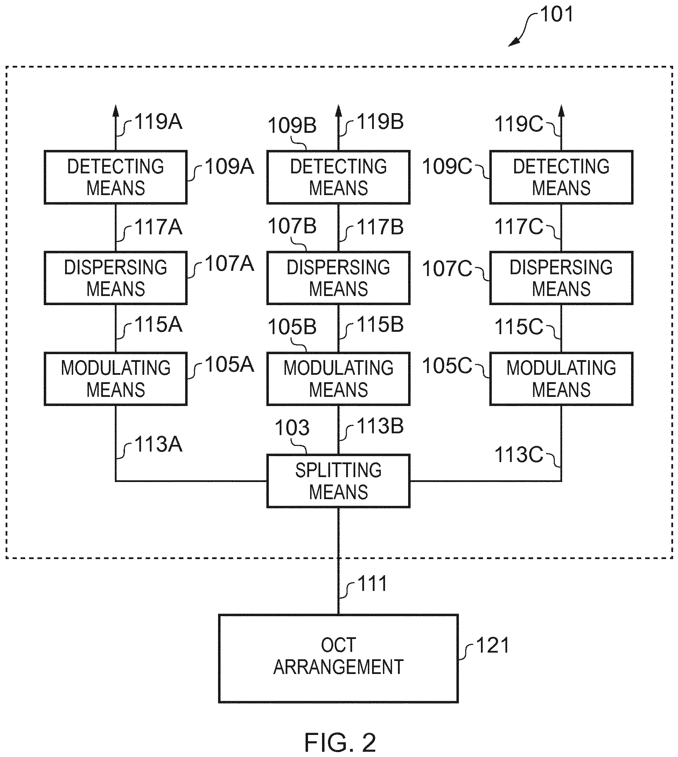

Fig. 2 illustrates another example apparatus 101 that could be provided in some examples of the disclosure. The example apparatus 101 shown in Fig. 2 is similar to the apparatus 101 shown in Fig. 1 except that the apparatus 101 shown in Fig. 2 comprises means 103 for splitting an input beam of light 111 into three beams of light. The apparatus 101 shown in Fig. 2 also comprises a plurality of means 105A, 105B, 105C for modulating beams of light, a plurality of means 107A for dispersing modulated beams of light and a plurality of means 109A, 109B, 109C for detecting dispersed beams of light. The plurality of means 105A, 105B, 105C for modulating beams of light, plurality of means 107A, 107B, 107C for dispersing modulated beams of light and plurality of means 109A, 109B, 109C for detecting dispersed beams of light could be as described in relation to Fig.1, corresponding reference numbers are used for corresponding features.

The splitting means 103 comprises any means which may be configured to split the input beam of light 111 into three separate beams of light. The separated beams of light can then be provided to three different channels. The splitting means 103 could comprise one or more beam splitters and/or any other suitable components.

In the example apparatus 101 shown in Fig. 2 the first beam of light 113A is provided to a first channel, the second beam of light 113B is provided to a second channel and the third beam of light 113C is provided to a third channel. Each of the first channel, the second channel and the third channel enable sparse sampling of the respective beam of light. Each of the first channel, the second channel and the third channel comprise modulating means 105A, 105B, 105C dispersing means 107A, 107B, 107C and detecting means 109A, 109B, 109C.

In the example apparatus 101 shown in Fig. 2 the first beam of light 113A from the splitting means 103 is provided to a first modulating means 105A to provide a first modulated beam of light 115A. The first modulated beam of light 115A is provided to a first dispersing means 107A to provide a first dispersed beam of light 117A. The first dispersed beam of light 117A is provided to the first detecting means 109A to provide a first output signal 119A. The second beam of light 113B from the splitting means 103 is provided to a second modulating means 105B to provide a second modulated beam of light 115B. The second modulated beam of light 115B is provided to a second dispersing means 107B to provide a second dispersed beam of light 117B. The second dispersed beam of light 117B is provided to the second detecting means 109B to provide a second output signal 119B. The third beam of light 113C from the splitting means 103 is provided to a third modulating means 105C to provide a third modulated beam of light 115C. The third modulated beam of light 115C is provided to a third dispersing means 107C to provide a third dispersed beam of light 117C. The third dispersed beam of light 117C is provided to the third detecting means 109C to provide a third output signal 119C.

The different dispersing means 107A, 107B, 107C in the different channels of the apparatus 101 may be configured to disperse the respective beams of light in different directions. In the example shown in Fig. 2 the first dispersing means 107A may be configured to disperse the light in a first direction, the second dispersing means 107B may be configured to disperse the light in a second, different direction. And the third dispersing means 107C may be configured to disperse the light in a third, different direction. In such examples the first direction could be at 60°, or substantially 60°, to the second direction and the second direction could be at 60°, or substantially 60°, to the third direction.

In the example apparatus 101 shown in Fig. 2 the first detector 109A provides a first output signal 119A, the second detector 109B provides a second output signal 119B and the third detector 109C provides the third output signal 119C. The output signals 119A, 119B, 119C each comprise information indicative of an object imaged by the OCT arrangement 121. However, the images that can be obtained from the output signals 119A, 119B, 119C may be different because the dispersing means 107A, 107B, 107C disperse the light in different directions. This means that different information could be comprised in the different output signals 119A, 119B, 119C.

In some examples the three output signals 119A, 119B, 119C can be combined to provide a single output signal which represents an image of the object imaged by the OCT arrangement 121. This image obtained using three different modulating and dispersing channels may be more accurate and may comprise more information than an image obtained using a single channel or two channels. The image could be rendered on a display or other suitable user output device.

In the example shown in Fig. 2 the splitting means 103 is configured to split the input beam of light 111 into three separate beams of light 113A, 113B, 113C. In other examples the splitting means 103 could be configured to split the input beam of light 111 into more than three separate beams of light which could be provided to more than three separate channels. In examples of the disclosure the different channels could be configured to modulate and detect the respective beams of light independently of the other channels within the apparatus 101. The number of channels that are provided within the apparatus 101 may depend on factors such as signal to noise ratios, the objects being imaged and any other suitable factors.

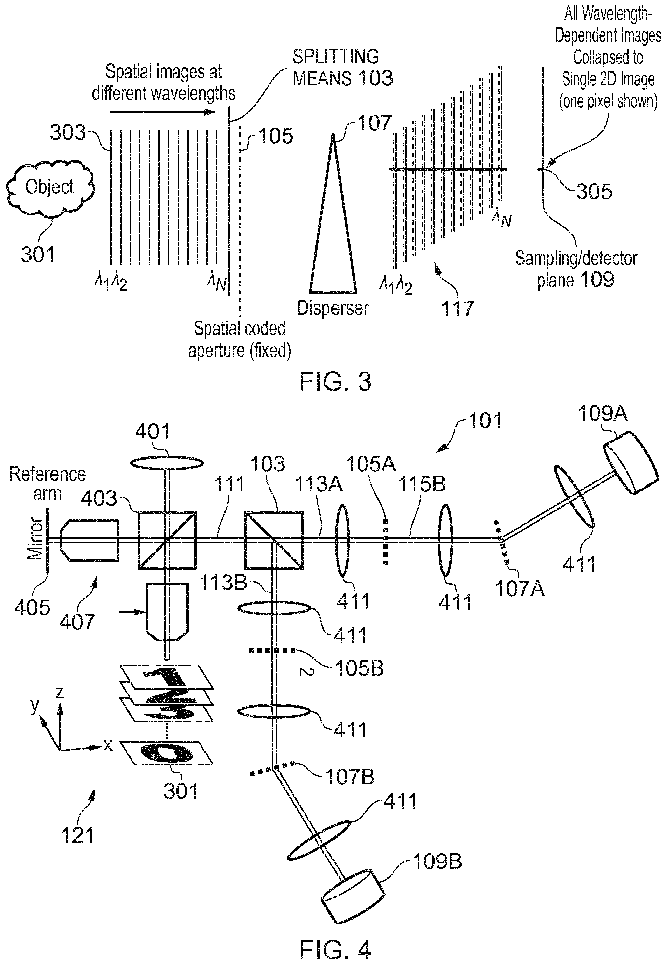

Fig. 3 illustrates an imaging principle of examples of the disclosure.

In the example of Fig. 3 an OCT arrangement (not shown for clarity) is used to image an object 301. The object 301 reflects light which has been directed onto the object 301. The object 301 could be part of a subject's body such as a retina or any other suitable type of object 301.

Different wavelengths of the incident light are reflected differently depending upon the internal structure of the object 301. This provides a plurality of spatial images 303. Each of the spatial images 303 corresponds to a different wavelength of light $\lambda_1$ to $\lambda_n$. The different spatial images 303 therefore comprise information about the internal structure of the object 301. The different spatial images 303 may comprise a three dimensional signal.

In the example of Fig. 3 the spatial images 303 are provided to splitting means. The splitting means splits the input beam of light 111 comprising the spatial images 303 into two or more beams of light. Each of the respective beams of light can then be provided from the beam splitter to a different channel of an apparatus 101. Only one channel is shown in Fig. 3. It is to be appreciated that the imaging principle would be the same for the other channels of the apparatus 101.

In the example of Fig. 3 the modulating means 105 comprises a two dimensional coded aperture. Other types of modulating means 105 may be used in other examples of the disclosure. In the example of Fig. 2 the modulating means 105 is fixed in position relative to the dispersing means 107 and the detecting means 109. In other examples the modulating means 105 could be moveable relative to the dispersing means 107 and the detecting means 109 and any other suitable components of the apparatus 101.

The spatial images 303 in the beam of light 113 provided by the splitting means 103 are modulated by the coded aperture of the modulating means 105. The coded aperture blocks and/or at least partially blocks portions of each of the different spatial images 303. The coded aperture may be wavelength dependent so that different spatial images 303 corresponding to different wavelengths may be blocked by different amounts.

The modulated beam of light 115 provided by the modulating means 105 is then spread by the dispersing means 107. In the example of Fig. 3 the dispersing means 107 comprises a prism. Other types of dispersing means 107 could be used in other examples of the disclosure. The dispersing means 107 refracts the modulated beam of light 115 to spatially spread the modulated beam of light 115. Different bandwidths of the spatial images 303 are spread by a different amount as shown schematically in Fig. 3. The distance by which a spatial image 303 is spread by the dispersing means 107 is dependent upon the wavelength of the spatial image 303.

The dispersed beam of light 117 is then incident upon the detecting means 109. The detecting means 109 comprises a plurality of pixels 305. Only one pixel 305 is shown for clarity in Fig. 3. The plurality of pixels 305 may be arranged in any suitable array. In the example of Fig. 3 the plurality of pixels 305 may be arranged in a matrix array comprising $N_x$ rows and $N_y$ columns. Each pixel 305 detects the summation of the dispersed beam of light 117 for each of the different wavelengths $\lambda_1$ to $\lambda_n$ for the area covered by the pixel 305.

As the different wavelengths $\lambda_1$ to $\lambda_n$ in the dispersed beam of light 117 are shifted by different amounts the different wavelengths $\lambda_1$ to $\lambda_n$ that are incident on a given pixel 305 of the detecting means 109 have passed though different portions of the modulating means 105. This means that the different wavelengths $\lambda_1$ to $\lambda_n$ that are incident on a given pixel 305 of the detecting means 109 may be modulated by different amounts.

In the above examples the beam of light 113 that is provided from the splitting means 103 to the modulating means 105 can be represented as $N_\lambda$ wavelength channels. Each of the wavelength channels has a spatial size $N_x \times N_y$.

The measurement Z obtained by the $(i,j)^{th}$ pixel where $Z \in \mathbb{R}^{N_x \times N_y}$ is given by equation 1

$$z(i,j) = \sum_{n_\lambda=1}^{N_\lambda} S_0(i,j,n_\lambda) M(i,j,n_\lambda). \tag{1}$$

Where $S_0(i,j,n_\lambda)$ is the three dimensional input signal and $M(i,j,n_\lambda)$ is a function representing the modulating means 105 and the dispersing means 107. The function $M(i,j,n_\lambda)$ will be dependent on the transparencies of the portions on the modulating means 105, the spatial arrangement of the portions of the modulating means 105, the dispersing means 107 and any other suitable factors.

Therefore in an apparatus 101 comprising two channels the measurement $Z_1$ obtained by the $(i,j)^{th}$ pixel of the first detecting means 107A is given by equation 2 and the measurement $Z_2$ obtained by the $(i,j)^{th}$ pixel of the second detecting means 107B is given by equation 3

$$z_1(i,j) = \sum_{n_\lambda=1}^{N_\lambda} S_0(i,j,n_\lambda) M_1(i,j,n_\lambda). \tag{2}$$

$$z_2(i,j) = \sum_{n_\lambda=1}^{N_\lambda} S_0(i,j,n_\lambda) M_2(i,j,n_\lambda). \tag{3}$$

The modulating means 105 may be represented as a matrix $\left\{ M^{(n_\lambda)} \right\}_{n_\lambda=1}^{N_\lambda} \in \mathbb{R}^{N_x \times N_y}$.

This allows the measurement Z obtained by each pixel 305 to be written in matrix form as

$$z = Hs, \tag{4}$$

where z is a vectorized version of the measurement obtained by each pixel 305, s is the stacked vector of the three dimensional input beam of light $S_0(x, y, \lambda)$ and $H \in \mathbb{R}^{(N_x N_y) \times (N_x N_y N_\lambda)}$ is the sensing matrix and can be represented by equation 5.

$$H = [Diag(M^{(1)}), \ldots Diag(M^{(N_\lambda)})] \tag{5}$$

In examples of the disclosure s is the spectral domain signal provided by an OCT arrangement. This allows equation (5) to be rewritten as

$$z = HFx \tag{6}$$

Where $x \in \mathbb{R}^{N_x N_y N_\lambda}$ denote the three dimensional image of the object and $F$ is the Fourier transform $F \in \mathbb{R}^{(N_x N_y N_\lambda) \times (N_x N_y N_\lambda)}$.

Therefore the measurement $z_1$ obtained by the first detecting means 109A and the measurement $z_2$ obtained by the second detecting means 109B can be written as:

$$z_1 = H_1 F_1 x \tag{7}$$

$$z_2 = H_2 F_2 x \qquad\qquad\qquad (8)$$

The image can therefore be obtained by solving

$$x = \arg \min_{x} \frac{1}{2} ||z_1 - H_1 F_1 x||_2^2 + \beta ||z_2 - H_2 F_2 x||_2^2 + \tau R(x) \qquad\qquad (9)$$

Where R(x) denotes the regularizer imposed on the OCT image $x$, and $\tau$ and $\beta$ balance the three terms in equations (9). For example, one choice for the value of $\beta$ would be $\frac{1}{2}$. Any suitable compressive sensing inversion algorithms may be used to solve equation (9) to obtain the desired image.

In this example only two detectors 109 are provided. It is to be appreciated that a similar equation could be solved where the apparatus 101 comprises more than two channels and more than two detectors 109 are provided.

Fig. 4 illustrates an OCT arrangement 121 and an apparatus 101. In the example shown in Fig. 4 the apparatus 101 comprises two channels and the splitting means 103 splits the input beam of light 111 into a first beam 113A and a second beam 113B. It is to be appreciated that in other examples the apparatus 101 could comprise more than two channels and the splitting means 103 could be configured to split the input beam of light 11 into more than two beams.

The OCT Arrangement 121 comprises a light source 401, a beam splitter 403, a static reference mirror 405 and one or more focusing elements 407. The OCT arrangement 121 shown in Fig. 4 is a spectral domain arrangement.

In examples of the disclosure the light source 401 is a broad beam light source which provides light having a range of wavelengths. The wavelength of the light that is used may depend on the type of object 301 that is to be imaged or any other suitable factor. In some examples the light used may be infrared light. In some examples the wavelength of the light used may be between 400nm to 1500nm.

The OCT arrangement 121 is configured so that the output light beam from the light source 401 is incident on the beam splitter 403. The beam splitter 403 may comprise a prism, a half silvered mirror or any other suitable component.

In the OCT arrangement 121 shown in Fig. 4 half of the split beam provides the reference beam and is provided to the static reference mirror 405. One or more focussing elements 407 are provided between the beam splitter 403 and the static reference mirror 405. The one or more focussing elements 407 may comprise any means which may be arranged to focus the beam of light. In some examples the one or more focussing elements 407 may comprise one or more lenses or any other suitable optical elements.

The other half of the split beam provides the object beam and is provided to the object 301. The object 301 may be arranged to be moved along the z axis. This axis may enable the focussing of the images provided by the OCT arrangement 121 and the apparatus 101. In the example of Fig. 4 the object 301 is provided on a motorised arrangement so as to enable movement along the z axis. In other examples a manual arrangement, or any other suitable type of arrangement, could be used.

One or more focussing elements 407 are provided between the beam splitter 403 and the object 301. The one or more focussing elements 407 may comprise any means which may be arranged to focus the beam of light. In some examples the one or more focussing elements 407 may comprise one or more lenses or any other suitable optical elements.

The different wavelengths of the light provide coherence of the object beam and the reference beam at different optical path lengths. Therefore the different wavelengths of light provide information about different depths within the object 301. Different features within the object 301 reflect the incident light by different amounts. The interference between the reflected object beam and the reflected reference beam therefore provides information about the features within the object 301.

As the different wavelengths of light provide information about different depths within the object 301 this enables three dimensional imaging of the object 301. The three dimensional imaging of the object 301 may enable different features at different depths within the object 301 to be identified and/or analysed. This ensures that the information obtained in the examples of the disclosure comprises information about the internal structure of an object 301 and not just information about the surface of the object 301.

The apparatus 101 is coupled to the OCT arrangement 121 so that the output beam of light from the OCT arrangement 121 is provided as an input beam of light 111 to the apparatus 101.

The input beam of light 111 is provided to the splitting means 103 so as to provide a first beam of light 113A and a second beam of light 113B.

The first beam of light 113A is provided to a first channel. The first channel comprises a first modulating means 105A, a first dispersing means 107A and a first detecting means 109A.

In the first channel a focussing element 411 is provided between the splitting means 103 and the first modulating means 105A. The focussing element 411 may comprise one or more lenses or any other suitable means that may be configured to focus the first beam of light 113A onto the first modulating means 105A.

The first modulating means 105A provides a first modulated beam of light 115A. A focussing element 411 is provided between the first modulating means 105A and the first dispersing means 107A. The focussing element 411 may comprise one or more lenses or any other suitable means that may be configured to focus the first modulated beam of light 115A onto the first dispersing means 107A.

The first dispersing means 107A provides a first dispersed beam of light 117A. In the example shown in Fig. 4 the first dispersing means 107A may be configured to disperse the light in a horizontal direction. The horizontal direction could be parallel, or substantially parallel, with the x axis as shown in Fig. 4.

A focussing element 411 is provided between the first dispersing means 107A and the first detecting means 109A. The focussing element 411 may comprise one or more lenses or any other suitable means that may be configured to focus the first dispersed beam of light 117A onto the first detecting means 109A.

The second beam of light 113B is provided to a second channel. The second channel comprises a second modulating means 105B, a second dispersing means 107B and a second detecting means 109B.

In the second channel a focussing element 411 is provided between the splitting means 103 and the second modulating means 105B. The focussing element 411 may comprise one or more lenses or any other suitable means that may be configured to focus the second beam of light 113B onto the second modulating means 105B.

The second modulating means 105B provides a second modulated beam of light 115B. A focussing element 411 is provided between the second modulating means 105B and the second dispersing means 107B. The focussing element 411 may comprise one or more lenses or any other suitable means that may be configured to focus the second modulated beam of light 115B onto the second dispersing means 107B.

The second dispersing means 107B provides a second dispersed beam of light 117B. In the example shown in Fig. 4 the second dispersing means 107B may be configured to disperse the light in a vertical direction. The vertical direction could be parallel, or substantially parallel, with the y axis as shown in Fig. 4. The vertical direction could be perpendicular, or substantially perpendicular, with the horizontal direction in which the first dispersing means 107A disperses the light in the first channel.

A focussing element 411 is provided between the second dispersing means 107B and the second detecting means 109B. The focussing element 411 may comprise one or more lenses or any other suitable means that may be configured to focus the second dispersed beam of light 117B onto the second detecting means 109B.

The output signal from the first detecting means 109A and the output signal from the second detecting means 109B may be combined to provide a combined image. The combined image may comprise more information than can be provided by a single channel.

In the example shown in Fig. 4 the first dispersing means 107A in the first channel disperse light in a direction which is perpendicular, or substantially perpendicular to the direction that the second dispersing means 107B in the second channel disperse the light. It is to be appreciated that this does not need to be the case in all examples of the disclosure. In other examples the different directions do not need to be perpendicular to each other.

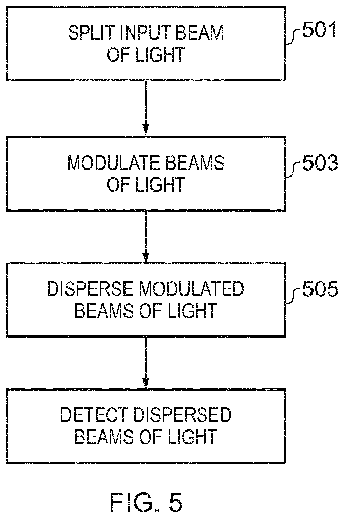

Fig. 5 illustrates an example method. The method may be implemented using any of the example apparatus 101 described above.

At block 501 the method comprises splitting an input beam of light 111. The input beam of light 111 is obtained from an OCT arrangement 121. The input beam of light 111 may be split into at least a first beam of light 113A and a second beam of light 113B.

At block 503 the method comprises modulating the beams of light 113A, 113B from the beam splitter. The first beam of light 113A is modulated to provide a first modulated beam of light 115A and the second beam of light 113B is modulated to provide a second modulated beam of light 115B.

At block 505 the method comprises dispersing the modulated beams of light 115A, 115B. The first modulated beam of light 115A is dispersed to provide a first dispersed beam of light 117A and the second modulated beam of light 115B is dispersed to provide a second dispersed beam of light 117B.

At block 507 the method comprises detecting the dispersed beams of light 117A, 117B and converting the detected beam of light into electrical output signals 119A, 119B.

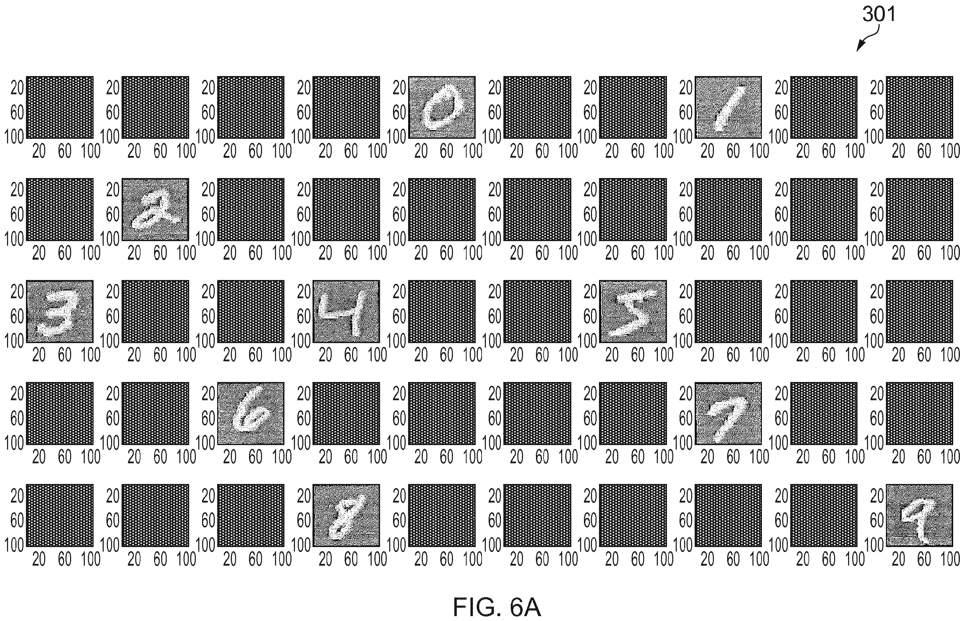

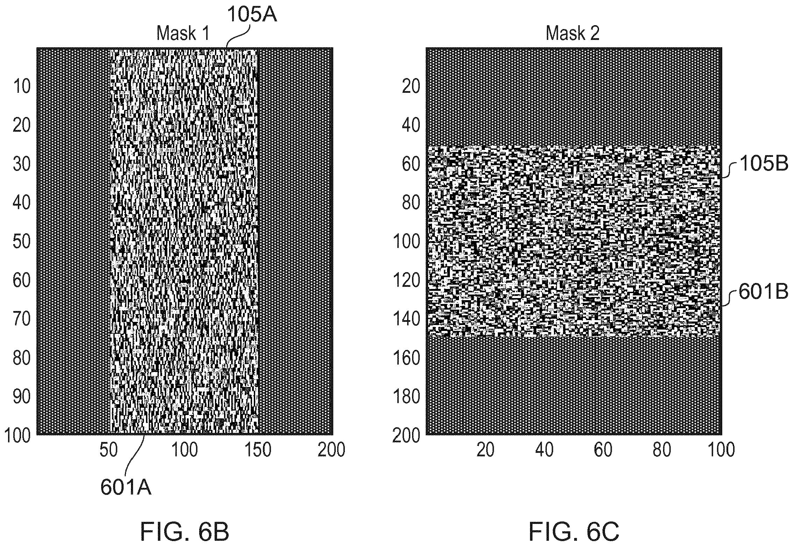

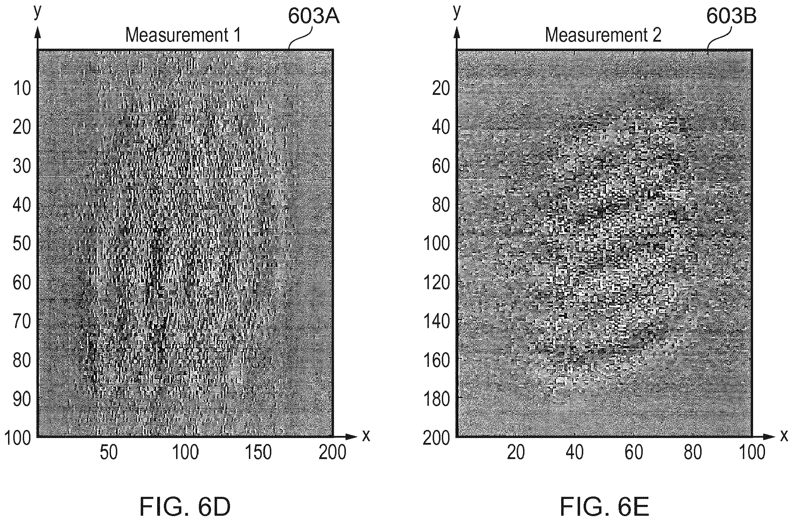

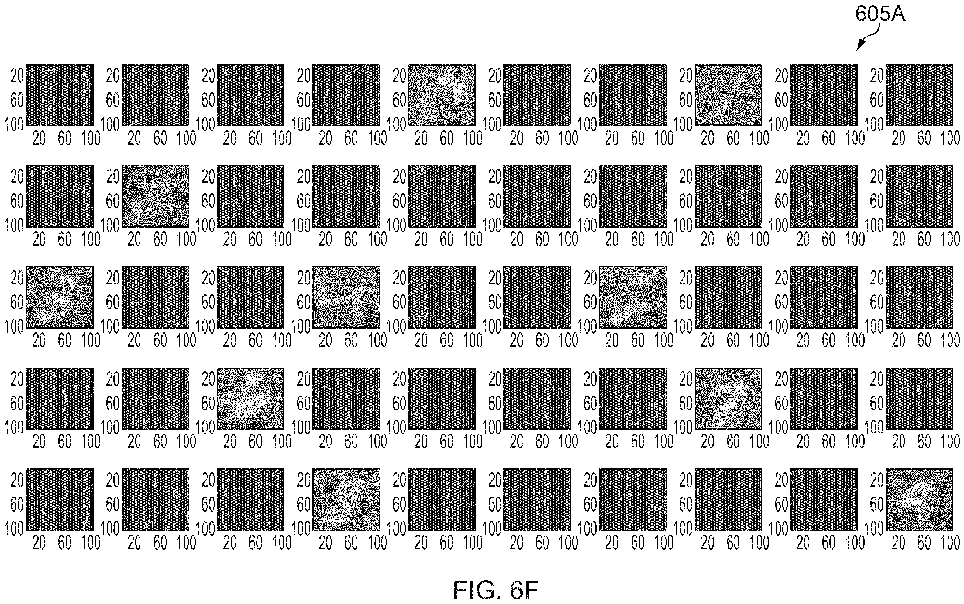

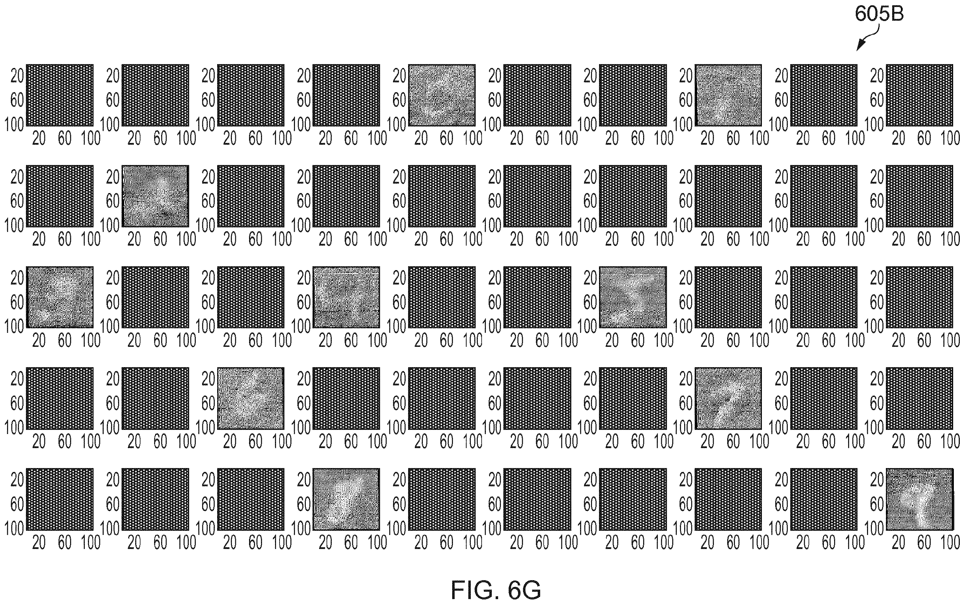

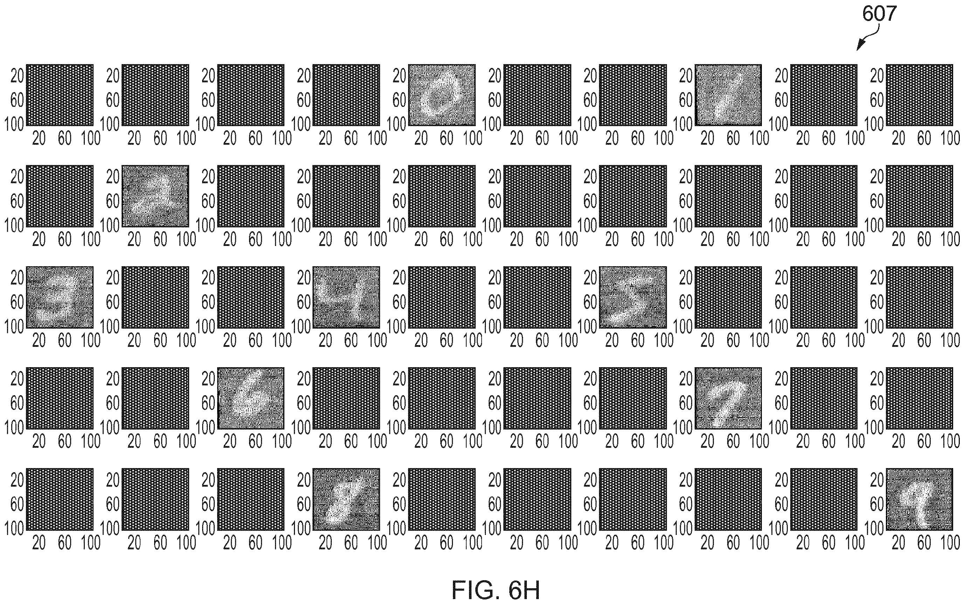

It is to be appreciated that in some examples the method may comprise further blocks that are not shown in Fig. 6. For instance, in some examples a modulating means 105 such as a coded aperture may be used to modulate the beams of light 113 and the method may comprise moving the modulating means so that different bandwidths are detected sequentially.

Figs. 6A to 6H illustrate example images that may be obtained using an example apparatus 101. In the examples shown in Figs. 6A to 6H the object 301 was imaged using a broadband light source with centre wavelength of 830nm and a bandwidth of 20nm. Other types of light sources could be used in other examples of the disclosure. In the examples shown in Figs. 6A to 6H the images are obtained using an apparatus 101 having two channels. It is to be appreciated that apparatus 101 comprising more channels could be used in other examples of the disclosure.

Fig. 6A illustrates an example object 301 that is imaged by the OCT arrangement. In the example of Fig. 6A the object is a three dimensional image array with dimensions of 100x100x50. Ten of the depths have a number on them as shown in Fig. 6A. Each of the frames shown in Fig. 6A represents a different depth of the image array.

Figs. 6B and 6C show masks 601A, 601B that can be used as modulating means 105A, 105B in examples of the disclosure. The first mask 601A may be provided as the first modulating means 105A in the first channel and the second mask 601 may be provided as the second modulating means 105B in the second channel.

In the examples shown in Figs. 6B and 6C the masks are binary masks. Each pixel within the masks has a value of 0 or 1 where 0 blocks the light and 1 enables passing of the light. It is to be appreciated that in some examples greyscale masks could be used where some of the pixels may enable part of the light to pass through.

In the examples shown in Figs. 6B and 6C the arrangement of the pixels in the masks 601, 601B is random or pseudo random. Other arrangements of the pixels could be used in other examples of the disclosure. The arrangement of the pixels in the masks 601A, 601B could be selected based on the types of objects 301 that are to be imaged or any other suitable factors. In such examples the pixels could be arranged in a customised pattern.

In the examples shown in Figs. 6B and 6C the first mask 601A is the same as the second mask 601B. However the second mask 601B has been rotated through 90° so that the second mask is perpendicular or substantially perpendicular to the first mask 601A. It is to be appreciated that other arrangements of the masks 601A, 601B could be used in other examples of the disclosure.

Figs. 6D and 6E show measurements that may be obtained by the detectors 109A, 109B of the apparatus 101. Fig. 6D shows an example measurement 603Athat may be obtained by the first detector 109A in the first channel and Fig. 6E shows an example measurement 603B that may be obtained by the second detector 109B in the second channel. In the first channel the dispersing means 107A has been configured to spread the light in a horizontal, or substantially horizontal, direction as indicated by the x axis in Fig. 6D. In the second channel the dispersing means 107B has been configured to spread the light in a vertical, or substantially vertical, direction as indicated by the y axis in Fig. 6E.

Fig. 6F shows a reconstructed image 605A of the object 301 that is obtained from the measurement obtained by the first detector 109A. The reconstructed image 605A may be obtained using the methods described above in relation to Fig. 3 or any other suitable method. Each of the frames shown in Fig. 6F show a different depth of the array of the object 301. In the example shown in Fig. 6F the peak-signal-to noise-ratio (PSNR) compared with truth is 23.1370dB.

Fig. 6G shows a reconstructed image 605B of the object 301 that is obtained from the measurement obtained by the second detector 109B. The reconstructed image 605B may be obtained using the methods described above in relation to Fig. 3 or any other suitable method. Each of the frames shown in Fig. 6G show a different depth of the array of the object 301. In the example shown in Fig. 6G the PSNR compared with truth is 23.0169dB.

Fig. 6H shows a reconstructed image 607 of the object 301 that is obtained from the both the measurements obtained by first detector 109A and the measurements obtained by the second detector 109B. The reconstructed image 6057 may be obtained by solving equation (9) as described above in relation to Fig. 3 or any other suitable method. Each of the frames shown in Fig. 6H show a different depth of the array of the object 301. In the example shown in Fig. 6H the PSNR compared with truth is 26.4347dB. Therefore the reconstructed image 607 that is obtained by combining measurements from two or more channels has a higher PSNR than the images obtained from single channels. Therefore the multi-channel apparatus 101 enables a higher quality image 607 to be obtained from the OCT arrangement 121.

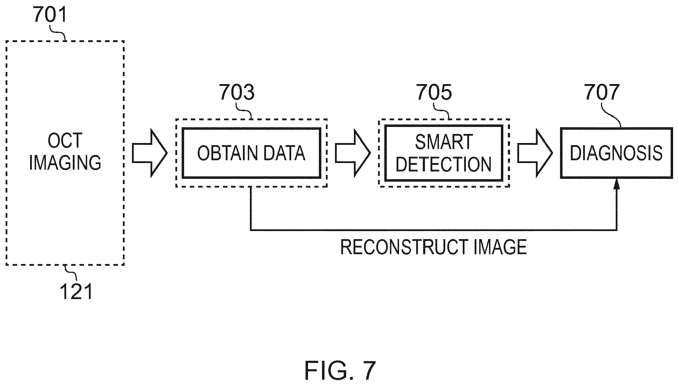

Fig. 7 illustrates a method of using examples of the disclosure which may be used to enable a medical diagnosis. At block 710 the OCT imaging is performed. The object 301 that is imaged may the retina of a person or animal or any other suitable object 301. The OCT imaging is performed by an OCT arrangement 121 which may be as described above.

At block 703 the data from the OCT imaging is obtained. The data is obtained by an apparatus 101 or plurality of apparatus 101 as described above. The apparatus 101 could comprise two or more different channels as described above. This enables a higher quality image to be obtained with a higher PSNR than an image obtained using a single channel.

The data obtained by the apparatus 101 or plurality of apparatus 101 is modulated by the modulating means 105. This enables the data to be captured in a compressed manner. This may provide for an efficient use of memory circuitry and communication bandwidths.

The obtained data is detected by the detecting means 109 as described above. The electrical output of the detecting means 109 may be used, at block 705 for smart detection. The smart detection may comprise the use of algorithms, or any other suitable technique, to recognise features in the output signal of the detecting means 109. This information could then be provided to the user, who may be a medical professional, at block 7077. The information that is provided may be used to enable a diagnosis by the medical professional.

In some examples, at block 707, the output signal from the detecting means 109 may be used to reconstruct an image of the object 301, or at least part of the object 301. This may also be provided to the medical professional to assist with any diagnosis.

The described examples therefore provide apparatus 101 comprising a plurality of channels which enable images with improved PSNRs to be obtained. The example apparatus 101 also provide for a fast capture of the information while providing for efficient use of communications bandwidths and memory circuitry.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting'.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

**Claims**

1. An apparatus comprising:

    means for splitting an input beam of light into at least a first beam of light and a second beam of light wherein the input beam of light is obtained from an optical coherence tomography arrangement;
    means for modulating the first beam of light to provide a first modulated beam of light and means for modulating the second beam of light to provide a second modulated beam of light;
    means for dispersing the first modulated beam of light to provide a first dispersed beam of light and means for dispersing the second modulated beam of light to provide a second dispersed beam of light; and
    means for detecting the first dispersed beam of light and means for detecting the second dispersed beam of light wherein the means for detecting the first dispersed beam of light and means for detecting the second dispersed beam of light are configured to convert the detected beams of light into electrical output signals.

**2.** An apparatus as claimed in claim 1 wherein the means for splitting an input beam of light into at least a first beam of light and a second beam of light are configured to split the input beam of light into more than two beams of light.

**3.** An apparatus as claimed in claim 2 comprising a means for modulating for each of the beams of light provided by the means for splitting an input beam of light, means for dispersing for each of the modulated beams of light and means for detecting for each of the dispersed beams of light.

**4.** An apparatus as claimed in any preceding claim wherein the means for modulating the beams of light comprise one or more coded apertures.

**5.** An apparatus as claimed in claim 4 wherein the one or more coded apertures comprise a two dimensional coded aperture.

**6.** An apparatus as claimed in any preceding claim wherein the means for modulating the beams of light comprises at least a first portion having a first transparency to the beam of light and at least a second portion having a second transparency to the beam of light, the second transparency being different from the first transparency.

**7.** An apparatus as claimed in claim 6 wherein the first transparency and the second transparency are wavelength dependent.

**8.** An apparatus as claimed in any of claims 6 to 7 wherein the portions of the means for modulating the input beam of light having different transparencies are arranged in a random pattern.

**9.** An apparatus as claimed in any preceding claim wherein the means for modulating the beams of light are arranged to convert a three dimensional signal into a two dimensional signal.

**10.** An apparatus as claimed in any preceding claim wherein the means for modulating the beams of light are arranged to be moveable relative to the means for dispersing the modulated beams of light and means for detecting the dispersed beams of light.

**11.** An apparatus as claimed in any preceding claim wherein the means for dispersing the modulated beams of light comprises at least one of; a prism, and a grating.

**12.** An apparatus as claimed in any preceding claim wherein the means for detecting the dispersed beams of light comprise at least one of a: charge coupled device, and a complementary metal-oxide semiconductor sensor.

**13.** An apparatus as claimed in any preceding claim wherein the means for detecting the dispersed beams of light comprise a two dimensional array of sensors.

**14.** An apparatus as claimed in any preceding claim wherein the optical coherence tomography arrangement is arranged so that the input beam of light comprises different wavelengths of light and the different wavelengths of light provide information about different depths within the object.

**15.** A method comprising:

splitting an input beam of light into at least a first beam of light and a second beam of light wherein the input beam of light is obtained from an optical coherence tomography arrangement;
modulating the first beam of light to provide a first modulated beam of light and modulating the second beam of light to provide a second modulated beam of light;
dispersing the first modulated beam of light to provide a first dispersed beam of light and dispersing the second modulated beam of light to provide a second dispersed beam of light; and
detecting the first dispersed beam of light and detecting the second dispersed beam of light and converting the detected beams of light into electrical output signals.

FIG. 1

101

119A
109B  119B
119C

| DETECTING MEANS | 109A | DETECTING MEANS | 109C | DETECTING MEANS |
|---|---|---|---|---|

117A
107B  117B
117C

| DISPERSING MEANS | 107A | DISPERSING MEANS | 107C | DISPERSING MEANS |
|---|---|---|---|---|

115A
105B  115B
115C

| MODULATING MEANS | 105A | MODULATING MEANS | 105C | MODULATING MEANS |
|---|---|---|---|---|

113A
103  113B
113C

| SPLITTING MEANS |
|---|

111

| OCT ARRANGEMENT | 121 |
|---|---|

FIG. 2

14

All Wavelength-
Dependent Images
Collapsed to
Single 2D Image
(one pixel shown)

Spatial images at
different wavelengths

SPLITTING
MEANS 103

303

Object

301

$\lambda_1\lambda_2$    $\lambda_N$

105

107

$\lambda_1\lambda_2$    $\lambda_N$

117

305

Disperser

Spatial coded
aperture (fixed)

Sampling/detector
plane 109

FIG. 3

101

109A

401

Reference
arm

403

Mirror

405

407

111

103

113A

113B

105A

115B

411

411

107A

411

411

411

105B

411

107B

411

109B

z

y

x

301

121

FIG. 4

```
┌─────────────────────────┐
│     SPLIT INPUT BEAM     │  ⌐501
│        OF LIGHT          │
└─────────────────────────┘
            │
            ▼
┌─────────────────────────┐
│     MODULATE BEAMS       │  ⌐503
│        OF LIGHT          │
└─────────────────────────┘
            │
            ▼
┌─────────────────────────┐
│   DISPERSE MODULATED     │  ⌐505
│     BEAMS OF LIGHT       │
└─────────────────────────┘
            │
            ▼
┌─────────────────────────┐
│   DETECT DISPERSED       │
│     BEAMS OF LIGHT       │
└─────────────────────────┘
```

FIG. 5

FIG. 6A

FIG. 6B

FIG. 6C

EP 3 653 987 A1

FIG. 6D

FIG. 6E

EP 3 653 987 A1

FIG. 6F

605A

FIG. 6G

FIG. 6H

FIG. 7

Europäisches
Patentamt

European
Patent Office

Office européen
des brevets

# EUROPEAN SEARCH REPORT

Application Number

EP 18 20 6841

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 3 477 246 A1 (NOKIA TECHNOLOGIES OY) 1 May 2019 (2019-05-01) * paragraph [0004] - paragraph [0018] * * paragraph [0020] - paragraph [0091]; claims 1-15; figures 1-7 * | 1-15 | INV. G01B9/02 A61B5/00 G01J3/28 G01J3/02 G01B11/24 |
| X | US 2011/285995 A1 (TKACZYK TOMASZ S [US] ET AL) 24 November 2011 (2011-11-24) | 1-6,9-15 | |
| Y | * paragraph [0057] - paragraph [0068] * * paragraph [0082] - paragraph [0087]; figure 13 * * paragraph [0099]; figure 22 * | 1-15 | |
| X | US 2016/265899 A1 (MINEMURA HIROYUKI [JP] ET AL) 15 September 2016 (2016-09-15) | 1,10,11, 14,15 | |
| Y | * paragraph [0076]; figure 1 * | 1-15 | |
| X | US 2015/063089 A1 (LIU SHANGQING [CA]) 5 March 2015 (2015-03-05) | 1-3,10, 11,13-15 | |
| Y | * paragraph [0043] - paragraph [0045]; figures 1-3 * | 1-15 | |
| X | Thuc-Uyen Nguyen: "Development of Hyperspectral Imagers for Snapshot Optical Coherence Tomography", , 25 April 2014 (2014-04-25), XP055407890, Retrieved from the Internet: URL:https://scholarship.rice.edu/bitstream /handle/1911/77422/NGUYEN-DOCUMENT-2014.pd f?sequence=1&isAllowed=y [retrieved on 2017-09-19] | 1-7,9-15 | TECHNICAL FIELDS SEARCHED (IPC) G01B G01J A61B |
| Y | * page 29 - page 36; figure 2 * | 1-15 | |

-/--

The present search report has been drawn up for all claims

| Place of search | Date of completion of the search | Examiner |
|---|---|---|
| Munich | 6 May 2019 | Braun, P |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

## EUROPEAN SEARCH REPORT

Application Number

EP 18 20 6841

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEÓN KARETH M ET AL: "Spectral dynamic scenes reconstruction based in compressive sensing using optical color filters", PROCEEDINGS OF SPIE; [PROCEEDINGS OF SPIE ISSN 0277-786X VOLUME 10524], SPIE, US, vol. 9860, 10 May 2016 (2016-05-10), pages 98600D-98600D, XP060068379, DOI: 10.1117/12.2224330 ISBN: 978-1-5106-1533-5 | 1-15 | |
| Y | * section 2; figures 1-3 * | 1-15 | |

----- 

TECHNICAL FIELDS SEARCHED (IPC)

The present search report has been drawn up for all claims

| Place of search | Date of completion of the search | Examiner |
|---|---|---|
| Munich | 6 May 2019 | Braun, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 20 6841

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2019

| Patent document cited in search report | | Publication date | Patent family member(s) | | Publication date |
|---|---|---|---|---|---|
| EP 3477246 | A1 | 01-05-2019 | EP | 3477246 A1 | 01-05-2019 |
| | | | WO | 2019081807 A1 | 02-05-2019 |
| US 2011285995 | A1 | 24-11-2011 | BR | PI0921770 A2 | 05-01-2016 |
| | | | CA | 2742273 A1 | 14-05-2010 |
| | | | CN | 102265124 A | 30-11-2011 |
| | | | EP | 2364436 A2 | 14-09-2011 |
| | | | JP | 2012508366 A | 05-04-2012 |
| | | | RU | 2011122642 A | 20-12-2012 |
| | | | US | 2011285995 A1 | 24-11-2011 |
| | | | WO | 2010053979 A2 | 14-05-2010 |
| US 2016265899 | A1 | 15-09-2016 | CN | 105973845 A | 28-09-2016 |
| | | | EP | 3067657 A1 | 14-09-2016 |
| | | | JP | 6462432 B2 | 30-01-2019 |
| | | | JP | 2016169948 A | 23-09-2016 |
| | | | US | 2016265899 A1 | 15-09-2016 |
| US 2015063089 | A1 | 05-03-2015 | NONE | | |

EPO FORM P0459

For more details about this annex : see Official Journal of the European Patent Office, No. 12/82